# EUROPÄISCHE PATENTSCHRIFT

(11) **EP 0 964 744 B1**
(45) Veröffentlichungstag und Bekanntmachung des Hinweises auf die Patenterteilung: **23.04.2003**
(21) Anmeldenummer: 98912298.1
(22) Anmeldetag: 12.02.1998
(51) Int. Cl.: B01J 23/22, B01J 23/04, C07C 51/255

(54) **VERFAHREN ZUR HERSTELLUNG VON PHTHALSÄUREANHYDRID UND TITAN-VANADIUM-CÄSIUM ENTHALTENDEM SCHALENKATALYSATOR HIERFÜR**
METHOD FOR PRODUCING PHTHALIC ANHYDRIDE; SHELL CATALYST CONTAINING TITANIUM-VANADIUM-CESIUM
PROCEDE DE PREPARATION D'ANHYDRIDE PHTALIQUE ET CATALYSEUR SOUS FORME DE COQUILLE CONTENANT TITANE-VANADIUM-CESIUM

(30) Priorität: 27.02.1997 DE 19707943
(43) Veröffentlichungstag der Anmeldung: 22.12.1999
(73) Patentinhaber: BASF AKTIENGESELLSCHAFT, 67056 Ludwigshafen (DE)
(72) Erfinder: HEFELE, Gerhard, D-67354 Römerberg (DE); KRATZER, Otto, D-67240 Bobenheim-Roxheim (DE); SCHEIDMEIR, Walter, D-67117 Limburgerhof (DE); ULRICH, Bernhard, D-67278 Bockenheim (DE)
(86) Internationale Anmeldenummer: EP9800779
(87) Internationale Veröffentlichungsnummer: WO98037965

(56) Entgegenhaltungen:
- EP-A- 0 021 325
- US-A- 4 046 780
- US-A- 4 879 387
- US-A- 5 169 820
- US-A- 5 235 071

## Beschreibung

Die vorliegende Erfindung betrifft ein Verfahren zur Herstellung von Phthalsäureanhydrid durch die katalytische Gasphasenoxidation von o-Xylol oder Naphthalin oder o-Xylol/Naphthalin-Gemischen mit einem molekularen Sauerstoff enthaltenden Gas und einem Schalenkatalysator aus einem inerten, nicht-porösen Trägermaterial auf das eine Titandioxid und Vanadiumpentoxid enthaltende katalytisch aktive Masse schichtförmig aufgebracht ist, sowie einen Katalysator hierfür.

Für die Herstellung von PSA aus o-Xylol oder Naphthalin oder o-Xylol/Naphthalin-Gemischen durch deren Oxidation mit molekularen Sauerstoff enthaltenden Gasen in der Gasphase an einem Festbettkatalysator wurden im Stand der Technik eine Vielzahl von katalysatoren vorgeschlagen. Die PSA-Katalysatoren bestehen im allgemeinen aus einem inerten Trägermaterial, auf das eine dünne Schicht der katalytisch aktiven Masse schalenförmig aufgetragen ist, weshalb diese Katalysatoren gemeinhin auch als Schalenkatalysatoren bezeichnet werden.

Für die katalytischen Eigenschaften dieser PSA-Katalysatoren spielt die Zusammensetzung der katalytisch aktiven Masse eine maßgebliche Rolle. Praktisch alle heutzutage genutzten PSA-Katalysatoren enthalten in ihrer katalytisch aktiven Masse die Komponenten Titandioxid, im allgemeinen in der Anatas-Modifikation, und Vanadiumpentoxid. Da PSA-Katalysatoren, deren katalytisch aktive Masse allein aus diesen Grundkomponenten besteht, hinsichtlich Umsatz, Ausbeute und Selektivität infolge von Nebenreaktionen, wie der Bildung von Phthalid der Formel

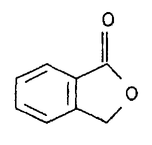

Maleinsäureanhydrid, Benzoesäure und Citraconsäureanhydrid der Formel 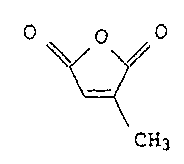 oder der Totalverbrennung, wirtschaftlich unbefriedigende Ergebnisse zeitigen und auch hinsichtlich Langzeitaktivität und -selektivität nicht zufriedenstellen können, gingen die Bestrebungen im Stand der Technik dahin, diese Katalysatoren durch Dotierung der katalytisch aktiven Masse mit den verschiedensten Zusatzstoffen und immer mehr Zusatzstoffen hinsichtlich ihrer Aktivität, Selektivität, Ausbeute und der Qualität des damit produzierten PSA-Produktes zu verbessern, d.h. es wurden mit der Zeit immer kompliziertere Katalysatorrezepte zur Lösung dieser Probleme entwickelt. Solche Zusatzstoffe sind z.B. Antimon, Bor, Cäsium, Calcium, Kobalt, Eisen, Kalium, Lithium, Molybdän, Natrium, Niob, Phosphor, Rubidium, Silber, Thallium, Wismut, Wolfram und Zinn.

In DE-A 24 36 009 und DE-A 24 21 406 werden Schalenkatalysatoren auf einem Steatitträger zur Herstellung von PSA verwendet, die in ihrer katalytisch aktiven Masse 60 bis 99 Gew.-% Titandioxid in der Anatas-Modifikation, 1 bis 40 Gew.-% Vanadiumpentoxid und bezogen auf das Titandioxid 0,15 bis 1,5 Gew.-% an Rubidium und/oder Cäsium enthalten.

DE-A 25 10 994 betrifft Vanadium- und Titan-haltige Trägerkatalysatoren einer bestimmten äußeren Form, die in ihrer katalytisch aktiven Masse 70 bis 99 Gew.-% Titandioxid in der Anatas-Modifikation und einer spezifischen inneren Oberfläche von 5 bis 20 m²/g, 1 bis 30 Gew.-% Vanadiumpentoxid und bis zu einem Anteil von 5 Gew.-% auch noch andere Stoffe, wie die Oxide der Elemente Cäsium, Rubidium, Thallium, Phosphor oder Antimon enthalten kann. Über den genauen Gehalt an diesen anderen Stoffen, insbesondere deren Mengenverhältnisse untereinander, werden keine Angaben gemacht. Als Trägermaterial wird Steatit (Magnesiumsilikat) verwendet.

In DE-A 25 47 624 werden Katalysatoren zur Herstellung von PSA beschrieben, die 60 bis 99 Gew.-% Titandioxid (Anatas), 1 bis 40 Gew.-% Vanadiumpentoxid und 0,1 bis 10 Gew.-% Rubidium und Antimon in einem Atomverhältnis Rb:Sb von 1:2,5 bis 1:30 in ihrer katalytisch aktiven Masse enthalten. Der verwendete Anatas kann danach eine innere Oberfläche von 5 bis 50, vorzugsweise 5 bis 20 m²/g haben, gemäß Ausführungsbeispiel wird Anatas mit einer inneren Oberfläche von 11 m²/g verwendet.

EP-A 21 325 betrifft Schalenkatalysatoren zur Herstellung von PSA, die in der katalytisch aktiven Masse 60 bis 99 Gew.-% Anatas, 1 bis 40 Gew.-% Vanadiumpentoxid und, bezogen auf die Gesamtmenge an -TiO₂ und V₂O₅, bis zu 2 Gew.-% Phosphor und bis zu 1,5 Gew.-% Rubidium und/oder Cäsium enthalten, wobei die katalytisch aktive Masse in zwei Schichten auf den Träger aufgetragen ist, deren innere Schicht 0,2 bis 2 Gew.-% Phosphor, aber kein Rubidium oder Cäsium und deren äußere Schicht 0 bis 0,2 Gew.-% Phosphor und 0,02 bis 1,5 Gew.-% Rubidium und/oder Cäsium enthält. Die katalytisch aktive Masse dieser Katalysatoren kann außer den genannten Bestandteilen noch kleine Mengen, z.B. bis zu 10 Gew.-% eines Oxids der Metalle Niob, Zinn, Silicium, Antimon, Hafnium, Molybdän oder Wolfram enthalten. Das zur Herstellung dieser Katalysatoren verwendete Titandioxid hat eine innere Oberfläche von 5 bis 30 m²/g. Als Trägermaterial wird Steatit verwendet.

EP-A 286 448 betrifft ein Verfahren zur Herstellung von PSA, bei dem zweierlei Katalysatoren in einer kombinierten Katalysatorschüttung eingesetzt werden, die beide ähnliche Gehalte an Titandioxid und Vanadiumpentoxid enthalten und sich im wesentlichen dadurch unterscheiden, daß der eine Katalysator zusätzlich 2 bis 5 Gew.-% einer Cäsiumverbindung, insbesondere Cäsiumsulfat, jedoch keine Phosphor-, Zinn-, Antimon-, Wismut-, Wolfram- oder Molybdänverbindungen enthält und der zweite Katalysator zusätzlich 0,1 bis 3 Gew.-% einer Phosphor-, Zinn-, Antimon-, Wismut-, Wolfram- oder Molybdänverbindung, jedoch praktisch kein Alkalimetall enthält.

EP-A 522 871, EP-A 539 878, EP-A 447 267, DE-A 29 48 163 und DE-A 30 45 624 betreffen allesamt Katalysatoren zur Herstellung von PSA, die aus einem porösen Trägermaterial, vorzugsweise Siliciumcarbid und einer katalytisch aktiven Masse, die neben Titandioxid udn Vanadiumpentoxid eine Vielzahl weiterer Katalytisch wirksamer Elemente, wie Phosphor, Alkalimetalle, Antimon (in EP-A 522 871 fünfwertiges Antimon), Niob und/oder Silber, enthält, zusammengesetzt sind.

Trotz Fortschritten in der Katalysator-Entwicklung sind die heute bekannten und verfügbaren Katalysatoren und Katalysatorsysteme für die Herstellung von Phthalsäureanhydrid weiterhin mit einer Reihe von Nachteilen behaftet. Die mit einem frischen Katalysator erzielbare anfängliche PSA-Ausbeute liegt bei etwa 80 mol-%, aber bereits im ersten Betriebsjahr ist ein deutlicher Ausbeuteabfall hinzunehmen. Aus Qualitätsgründen muß das zunächst als Rohware anfallende Phthalsäureanhydrid einer chemischen Behandlung unterworfen werden, ehe es anschließend destillativ zu Reinprodukt im heute geforderten Qualitätsstandard aufgearbeitet werden kann. Wegen ihrer großen Empfindlichkeit gegenüber Temperatur-, Druckund Beladungsschwankungen ist für die sichere Nutzung dieser Katalysatoren im Großbetrieb ein hoher Überwachungs- und Kontrollaufwand erforderlich. Ein weiterer Nachteil ergibt sich für diese Katalysatoren dadurch, daß aufgrund ihres unvollständigen o-Xylol-Umsatzes und dem Entstehen von unter- bzw. überoxidierten Nebenprodukten Geruchsprobleme sowie o-Xylol- und Benzolemissionen entstehen, die aus Umweltgründen eine aufwendige Abgasverbrennung erforderlich machen.

Diese Nachteile verstärken sich bei hohen Beladungen des Eduktgasstroms mit o -Xylol und/oder Naphthalin, insbesondere bei Beladungen von 80 g o-Xylol je Nm³ Gas und mehr.

Der vorliegenden Erfindung lag daher die Aufgabe zugrunde, ein Verfahren zur Herstellung von PSA zu finden, das frei von den obengenannten Nachteilen ist und hierfür einen geeigneten Katalysator zur Verfügung zu stellen. Das erfindungsgemäße Verfahren sollte insbesondere im Langzeitbetrieb mit hohen o-Xylolbeladungen dauerhaft gute Ausbeuten von PSA mit einem hohen Reinheitsgrad liefern und so eine chemische Aufarbeitung des rohen PSA zur Abtrennung nachteiliger, aber destillativ praktisch kaum abtrennbarer Nebenprodukte, wie Phthalid, überflüssig machen.

Dementsprechend wurde ein Verfahren zur Herstellung von Phthalsäureanhydrid durch die katalytische Gasphasenoxidation von o-Xylol oder Naphthalin oder o-Xylol/Naphthalin-Gemischen mit einem molekularen Sauerstoff enthaltenden Gas an einem Schalenkatalysator aus einem inerten, nicht-porösen Trägermaterial, auf das eine Titandioxid und Vanadiumpentoxid enthaltende katalytisch aktive Masse schichtförmig aufgebracht ist, gefunden, das dadurch gekennzeichnet ist, daß man einen Katalysator dessen katalytisch aktive Masse zu 3 bis 6 Gew.-% aus Vanadiumpentoxid, berechnet als V₂O₅, zu 0,3 bis 0,5 Gew.-% aus einer Cäsiumverbindung, berechnet als Cs, und der Rest zu 100 Gew.-% aus Titandioxid in der Anatasmodifikation besteht, in An- oder Abwesenheit eines von diesem verschiedenen Schalenkatalysator zur katalytischen Gasphasenoxidation von o-Xylol oder Naphthalin oder o-Xylol/Naphthalin-Gemischen, verwendet und bei Anwesenheit eines solchen zweiten Katalysators, diesen in einer kombinierten Schüttung mit dem Katalysator obenstehender Zusammensetzung im Reaktor einsetzt.

Insbesondere wurde ein Verfahren zur Herstellung von Phthalsäureanhydrid durch die katalytische Gasphasenoxidation von o-Xylol oder Naphthalin oder o-Xylol/Naphthalin-Gemischen mit einem molekularen Sauerstoff enthaltenden Gas an einem Schalenkatalysator aus einem inerten, nicht-porösen Trägermaterial auf das eine Titandioxid und Vanadiumpentoxid enthaltende katalytisch aktive Masse schichtförmig aufgebracht ist, gefunden, das dadurch gekennzeichnet ist, daß man in einer ersten zum Gaseintritt in den Reaktor hin gelegenen Reaktionszone einen Schalenkatalysator einsetzt, dessen katalytisch aktive Masse 3 bis 6 Gew.-% aus Vanadiumpentoxid, berechnet als V₂O₅, zu 0,3 bis 0,5 Gew.-% aus einer Cäsiumverbindung, berechnet als Cs und der Rest zu 100 Gew.-% aus Titandioxid in der Anatas-Modifikation besteht und einer zweiten zum Austritt der Reaktionsgase aus dem Reaktor hin gelegenen Reaktionszone einen Schalenkatalysator verwendet, dessen katalytisch aktive Masse zu 1 bis 10 Gew.-% aus Vanadiumpentoxid, berechnet als V₂O₅, zu 0 bis 10 Gew.-% aus Antimonoxid, berechnet als Sb₂O₃, zu 0,01 bis 0,3 Gew.-% aus einer Cäsium- und/oder Rubidiumverbindung, berechnet als Cs bzw. Rb, zu 0,01 bis 0,3 Gew.-% aus einer Phosphorverbindung, berechnet als P, und im Rest zu 100 Gew.-% aus Titandioxid in der Anatas-Modifikation besteht.

Desweiteren wurde ein Katalysator aus einer dünnen Schicht katalytisch aktiver Komponenten, die auf einem nicht-porösen Trägermaterial schalenförmig aufgebracht ist, dessen katalytisch aktive Masse zu 3 bis 6 Gew.-% aus Vanadiumpentoxid, berechnet als V₂O₅, zu 0,3 bis 0,5 Gew.-% aus einer Cäsiumverbindung, berechnet als Cs, und der Rest zu 100 Gew.-% aus Titandioxid in der Anatas-Modifikation besteht, gefunden.

Der erfindungsgemäße Katalysator kann allein oder vorzugsweise in einer kombinierten Schüttung mit einem zweiten oder mehreren davon verschiedenen Katalysatoren im erfindungsgemäßen Verfahren eingesetzt werden. Bevorzugt wird der erfindungsgemäße Katalysator in einer kombinierten Schüttung mit einem zweiten Katalysator eingesetzt, insbesondere mit einem zweiten Katalysator der zuvor beschriebenen Zusammensetzung.

Das erfindungsgemäße Verfahren wird somit vorzugsweise unter Anwendung einer kombinierten Katalysatorschüttung ausgeführt, d.h. die beiden erfindungsgemäß verwendeten Katalysatoren werden in den einzelnen Reaktionsrohren des PSA-Reaktors in einem Festbett übereinander angeordnet, so daß der erste Katalysator zum Gaseintritt des Eduktgasstroms in den Reaktor hin gelegen ist, wohingegen der zweite Katalysator zum Gasaustritt der Reaktionsgase aus dem Reaktor hin gelegen ist. Da die Umsetzung des aromatischen Kohlenwasserstoffs in der Katalysatorschüttung stattfindet, wird der mit den betreffenden Katalysatoren befüllte Abschnitt des oder der Reaktionsrohre auch als Reaktionszone und der mit dem ersten bzw. zweiten Katalysator befüllte Abschnitt der Reaktionszone auch als erste bzw. zweite Reaktionszone bezeichnet. Je nach Art der erfindungsgemäß verwendeten PSA-Katalysatoren kann der Anteil der ersten Reaktionszone am Volumen der genannten Reaktionszone zwischen 25 und 75 Vol.-%, vorzugsweise zwischen 50 und 70 Vol.-% liegen.

In der ersten Reaktionszone wird erfindungsgemäß vorteilhaft ein Schalenkatalysator verwendet, auf dessen inertes, nicht-poröses unter den Bedingungen der PSA-Herstellung hitzebeständiges Trägermaterial die katalytisch aktive Masse in einer schalenförmigen Schicht aufgebracht ist, wobei diese katalytisch aktive Masse, bezogen auf das Gewicht dieser katalytisch aktiven Masse im fertigen Katalysator, im allgemeinen 3 bis 6 Gew.-%, vorzugsweise 3 bis 5 Gew.-% und besonders bevorzugt 3,5 bis 4,5 Gew.-% Vanadiumpentoxid, berechnet als V₂O₅, sowie im allgemeinen 0,3 bis 0,5 Gew.-%, bevorzugt 0,35 bis 0,45 Gew.-% einer Cäsiumverbindung, berechnet als Cs, enthält und der Rest zu 100 Gew.-% der katalytisch aktiven Masse aus Titandioxid in dessen Anatas-Modifikation besteht. Da nicht bekannt ist, in Form welcher Verbindung oder Verbindungen das Cäsium im fertigen Katalysator vorliegt, wird der Gehalt an Cäsiumverbindungen als Cs berechnet.

Bei der Angabe dieser Zusammensetzung, die die aktiv zur Herstellung der katalytisch aktiven Masse zugesetzten Komponenten umfaßt, wurden gegebenenfalls in den Ausgangs- bzw. Vorläuferverbindungen als technisch unvermeidbare Verunreinigungen enthaltene Elemente nicht berücksichtigt, da diese nicht für jeden Bestandteil und nicht in jedem Versuch analysiert wurden.

Als inertes, nicht-poröses und unter den Bedingungen der PSA-Herstellung hitzebeständiges Trägermaterial kann der Katalysator gesinterte oder aufgeschmolzene Silikate, z.B. Steatit (Magnesiumsilikat), Porzellan, Tonerde, nicht-poröses Siliciumcarbid, Rutil oder Quarz enthalten, bevorzugt ist das Trägermaterial im erfindungsgemäß eingesetzten Katalysator Steatit. Das Trägermaterial kann zur Beschichtung mit der katalytisch aktiven Masse z.B. in Gestalt von Kugeln, Zylindern, Spiralen oder Ringen eingesetzt werden, vorzugsweise wird ein Träger in Form von Ringen, wie in DE-A 25 10 994 beschrieben, verwendet. Im fertigen Katalysator nimmt die katalytisch aktive Masse, bezogen auf das Gesamtgewicht des Katalysators, im allgemeinen einen Anteil von 8 bis 12 Gew.-%, vorzugsweise von 9 bis 11 Gew.-% und besonders bevorzugt von 9,5 bis 10,5 Gew.-% ein. Unter "fertigen Katalysator" wird in dieser Anmeldung der mit der katalytisch aktiven Masse beschichtete, gegebenenfalls zur Überführung von Vorläuferverbindungen der katalytisch aktiven Komponenten in diese katalytisch aktiven Komponenten und zur Entfernung organischer Hilfsmittel zur Katalysatorherstellung wärmebehandelte, einsatzbereite Katalysator verstanden.

Zur Herstellung des erfindungsgemäß anzuwendenden Katalysators wird Titandioxid in der Anatas-Modifikation verwendet, das im allgemeinen eine BET-Oberfläche von 13 bis 28 m²/g, besonders bevorzugt von 19 bis 21 m²/g und eine Korngröße von im allgemeinen 0,12 bis 1 µ, vorzugsweise von 0,4 bis 0,8 µ hat.

Der in der zweiten Reaktionszone erfindungsgemäß zu verwendende Katalysator ist vom Katalysator der ersten Reaktionszone verschieden. Besonders bevorzugt wird in der zweiten Reaktionszone ein Katalysator eingesetzt, der in seiner katalytisch aktiven Masse, bezogen auf das Gesamtgewicht der katalytisch aktiven Masse im fertigen Katalysator, im allgemeinen 1 bis 10 Gew.-%, vorzugsweise 2 bis 9 Gew.-% und besonders bevorzugt 3 bis 8 Gew.-% Vanadiumpentoxid, berechnet als V₂O₅, im allgemeinen 0 bis 10 Gew.-%, vorzugsweise 0 bis 5 Gew.-% und besonders bevorzugt 0 bis 4 Gew.-% Antimonoxid, berechnet als Sb₂O₃, im allgemeinen 0,01 bis 0,3 Gew.-%, vorzugsweise 0,05 bis 0,3 Gew.-% und besonders bevorzugt 0,1 bis 0,25 Gew.-% einer Cäsium- und/oder Rubidiumverbindung, berechnet als Cs bzw. Rb, sowie im allgemeinen 0,01 bis Q,3 Gew.-%, vorzugsweise 0,05 bis 0,3 Gew.-% und besonders bevorzugt 0,1 bis 0,25 Gew.-% einer Phosphorverbindung, berechnet als P, enthält. Der Gehalt an einer Phosphorverbindung im Katalysator wird als P berechnet, da nicht bekannt ist, in Form welcher Verbindung oder Verbindungen die bei der Herstellung zugesetzte Phosphorverbindung oder zugesetzten Phosphorverbindungen im fertigen Katalysator vorliegen.

Selbstverständlich können in der zweiten Reaktionszone auch andere zur Oxidation von aromatischen Kohlenwasserstoffen geeignete Katalysatoren mit einer von der vorstehenden Zusammensetzung verschiedenen Zusammensetzung eingesetzt werden, beispielsweise mit im Handel erhältlichen Katalysatoren oder Katalysatoren wie sie z.B. in DE-A 25 46 268, EP-A 286 448, DE-A 25 47 624, DE-A 29 48 163, EP-A 163 231, DE-A 28 30 765, DE-A 17 69 998, EP-A 522 871, EP-A 539 878, EP-A 447 267, DE-A 30 45 624 oder DE-A 40 13 051 beschrieben sind.

Die bei den Erläuterungen des in der ersten Reaktionszone erfindungsgemäß vorteilhaft zu verwendenden Katalysators gegebenen Angaben zum Trägermaterial, zur Art des für die katalytisch aktive Masse zu verwendenden Titandioxids und zum Gewichtsanteil der katalytisch aktiven Masse am Gesamtkatalysator, gelten auch für den in der zweiten Reaktionszone erfindungsgemäß vorteilhaft einzusetzenden Katalysator.

Entsprechend den vorstehenden Angaben zur Zusammensetzung des in der zweiten Reaktionszone vorzugsweise einzusetzenden Katalysators kann dieser Antimon enthalten oder Antimon-frei sein. Das Antimonoxid kann in diesen Katalysatoren z.B. als Sb₂O₃, Sb₂O₄ oder Sb₂O₅ vorliegen, bevorzugt wird Sb₂O₃ zur Herstellung des Katalysators verwendet.

Die Angaben zur Zusammensetzung des in der zweiten Reaktionszone vorzugsweise verwendeten Katalysators geben die Zusammensetzung der katalytisch aktiven Masse also der Element-Komponenten an, die aktiv zu deren Herstellung eingesetzt wurden. Eventuell in den Ausgangsmaterialien zur Herstellung der katalytisch aktiven Masse enthaltene, technisch unvermeidbare Verunreinigungen wurden bei diesen Angaben nicht berücksichtigt, da diese nicht für jeden Bestandteil und nicht in jedem Versuch analysiert wurden.

Die Herstellung der in den beiden Reaktionszonen erfindungsgemäß vorteilhaft anzuwendenden Katalysatoren kann auf an sich herkömmliche Weise durch Aufbringen der katalytisch aktiven Masse oder von Vorläuferverbindungen der in der katalytisch aktiven Masse enthaltenen katalytisch aktiven Komponenten auf den Träger erfolgen, beispielsweise wie in DE-A 25 10 994 beschrieben, durch Aufsprühen einer die Komponenten der katalytisch aktiven Masse oder deren Vorläuferverbindungen enthaltenden Maische auf den z.B. auf 100 bis 450°C vorerwärmten Träger, beispielsweise in einer Dragiertrommel. Die katalytisch aktive Masse kann auch anders als durch Aufsprühen, z.B. durch Auftragen einer die katalytisch aktiven Komponenten oder deren Vorläuferverbindungen sowie Hilfsmittel zur Katalysatorherstellung enthaltenden pastösen Masse auf den Träger, z.B. in einer Dragiertrommel, oder durch Auftragen eines die katalytisch aktiven Komponenten und/oder deren Vorläuferverbindungen enthaltenden, z.B. durch Sprüh- oder Gefriertrocknung vorgefertigten Pulvers auf das Trägermaterial, z.B. in einer Dragiertrommel, wobei während des Beschichtungsvorgangs eine geringe Menge eines Lösungsmittel zur Vermittlung der Haftung des Pulvers auf dem vorerwärmten Träger in die Beschichtungsvorrichtung eingesprüht wird, anschließende Trocknung und gegebenenfalls Calcinierung bei Temperaturen bis 450°C, vorzugsweise bis 400°C, auf das Trägermaterial aufgebracht werden.

Zur Herstellung der Maische bzw. der pastösen Masse werden die katalytisch aktiven Komponenten des Katalysators bzw. deren Vorläuferverbindungen beispielsweise in Form ihrer Oxide, Salze, ihrer Nitrate, C₁- bis C₁₀-carboxylate, Carbonate, Hydrogencarbonate, Sulfate, Hydrogensulfate, Halogenide oder Phosphate oder als Komplexverbindungen, beispielsweise als Oxalat- oder Acetylaceton-Komplexe sowie gegebenenfalls zur Katalysatorherstellung eingesetzte Hilfsmittel in einem Lösungsmittel gelöst oder, falls diese Stoffe im einzelnen nicht löslich sind, suspendiert. Als Lösungs- oder Suspendierungsmittel können sowohl Wasser als auch organische Flüssigkeiten oder Mischungen des Wassers mit diesen Flüssigkeiten verwendet werden. Vorzugsweise wird Wasser oder Wasser im Gemisch mit organischen Flüssigkeiten verwendet, wobei das Mischungsverhältnis Wasser/organische Flüssigkeit im allgemeinen zwar nicht kritisch ist, vorzugsweise aber solche Lösungsmittelgemische eingesetzt werden, die 50 oder mehr Gewichtsprozent Wasser enthalten.

Als organische Flüssigkeiten werden vorzugsweise wasserlösliche Lösungsmittel, wie C₁- bis C₄-Alkohole, wasserlösliche Ether, z.B. Tetrahydrofuran, Dioxan oder Ethylenglykoldimethylether, wasserlösliche Amide, z.B. Formamid, Pyrrolidon, N-Methylpyrrolidon, N,N-Dimethylformamid oder wasserlösliche Sulfoxide, z.B. Dimethylsulfoxid, verwendet.

Als Hilfsmittel für die Katalysatorherstellung können der Maische bzw. der pastösen Masse Bindemittel, Porenbildner und/oder temporäre Aktivitätsdämpfungsmittel zugesetzt werden.

Unter dem Begriff Bindemittel werden hierbei Substanzen verstanden, die die Haftung der einzelnen Partikel der katalytisch aktiven Masse oder deren Vorläuferverbindungen untereinander und/oder auf dem Trägermaterial dauerhaft oder temporär verbessern. Zur Herstellung der erfindungsgemäß zu verwendenden Katalysatoren können als Bindemittel beispielsweise Polyole, wie Ethylenglykol, Propylenglykol, Butylenglykole oder Glycerin oder Amide, wie Formamid, N,N-Dimethylformamid, N,N-Diethylformamid, N,N-Dibutylformamid, Acetamid, Pyrrolidon oder N-Methylpyrrolidon verwendet werden.

Als Porenbildner werden im folgenden Substanzen bezeichnet, die in der Aktivmasse durch eine Volumenänderung, beispielsweise durch Verdampfung oder Zersetzung während der Wärmebehandlung zur Erzeugung der katalytisch aktiven Metalloxide aus deren Vorläuferverbindungen, bei der Herstellung des Schalenkatalysators, die Herausbildung einer im Vergleich zu einer Aktivmasse, bei deren Herstellung kein Porenbildner zugesetzt wurde, geänderte Porenstruktur bewirken. Als Porenbildner können im erfindungsgemäßen Verfahren beispielsweise Polyole, wie Glycerin oder Polymere, wie Cellulose, Methylcellulose, Celluloseacetat oder Stärke, oder Säuren wie Oxalsäure, Weinsäure oder Milchsäure, oder auch Amine, wie Melamin, oder Amide, wie Harnstoff, eingesetzt werden. Die Art und die Menge der zuzusetzenden Hilfsmittel richtet-sich im allgemeinen nach der chemischen Zusammensetzung der katalytisch aktiven Masse des betreffenden Schalenkatalysators und der verwendeten Ausgangsmaterialien und wird zweckmäßigerweise für die jeweils zu erzeugende katalytisch aktive Masse bestimmter chemischer Zusammensetzung in einem Vorversuch optimiert.

Unter dem Begriff temporäre Aktivitätsdämpfungsmittel werden oben angegebene Bindemittel oder Porenbildner oder darüber hinaus auch alle weiteren Hilfsstoffe verstanden, die einen begrenzten Zeitraum zu einer Aktivitätsverminderung und/oder zu einer Verringerung der hot spot-Temperatur führen und somit das Anfahren des Reaktors bzw. die Beladungserhöhung bis zu dessen Vollastbetrieb erleichtern ohne die mittlere katalytische Aktivität bzw. Selektivität zu vermindern.

Die so hergestellten Katalysatoren werden vorzugsweise zur Gasphasenoxidation von o-Xylol oder Naphthalin oder o-Xylol/Naphthalin-Gemischen zu Phthalsäureanhydrid verwendet.

Zu diesem Zweck werden die erfindungsgemäß anzuwendenden Katalysatoren in die Reaktionsrohre gefüllt, die dann vorteilhaft von außen, beispielsweise mittels eines Salzbades, auf die Reaktionstemperatur thermostatisiert werden. Über die so bereitete Katalysatorschüttung wird das Reaktionsgas bei Temperaturen von im allgemeinen 300 bis 450°C, vorzugsweise von 320 bis 420°C und besonders bevorzugt von 340 bis 400°C und bei einem Überdruck von im allgemeinen 0,1 bis 2,5 bar, vorzugsweise von 0,3 bis 1,5 bar mit einer Raumgeschwindigkeit von im allgemeinen 750 bis 5000 h⁻¹ geleitet.

Das dem Katalysator zugeführte Reaktionsgas wird im allgemeinen durch Vermischen von einem molekularen Sauerstoff enthaltenden Gas, vorzugsweise Luft, das außer Sauerstoff noch geeignete Reaktionsmoderatoren und/oder Verdünnungsmittel, wie Dampf, Kohlendioxid und/oder Stickstoff, enthalten kann, mit dem zu oxidierenden, aromatischen Kohlenwasserstoff erzeugt, wobei das molekularen Sauerstoff enthaltende Gas im allgemeinen 1 bis 100 Vol-%, vorzugsweise 2 bis 50 Vol-% und besonders bevorzugt 10 bis 30 Vol-% Sauerstoff, 0 bis 30 Vol-%, vorzugsweise 0 bis 10 Vol-% Wasserdampf sowie 0 bis 50 vol-%, vorzugsweise 0 bis 1 Vol-% Kohlendioxid, Rest Stickstoff, enthalten kann. Zur Erzeugung des Reaktionsgases wird das molekularen Sauerstoff enthaltende Gas im allgemeinen mit 40 g bis 140 g je Nm³ Gas, vorzugsweise mit 60 bis 120 g je Nm³ Gas und besonders bevorzugt mit 80 bis 115 g je Nm³ Gas des zu oxidierenden, aromatischen Kohlenwasserstoffs beschickt.

Vorteilhaft kann die Gasphasenoxidation so durchgeführt werden, daß man zwei oder mehr Zonen, vorzugsweise zwei Zonen der im Reaktionsrohr befindlichen Katalysatorschüttung auf unterschiedliche Reaktionstemperaturen thermostatisiert, wobei beispielsweise Reaktoren mit getrennten Salzbädern, wie sie z.B. in DE-A 22 01 528 oder DE-A 28 30 765 beschrieben sind, eingesetzt werden können. Dabei kann, wie in DE-A 40 13 051 beschrieben, die zum Gaseintritt des Eduktgases hin gelegene Reaktionszone, welche, wie bereits erwähnt, im allgemeinen 25 bis 75 Vol-% des gesamten Katalysatorvolumens umfaßt, auf eine um 1 bis 20°C, vorzugsweise 1 bis 10°C und insbesondere um 2 bis 8°C höhere Reaktionstemperatur als die zum Gasaustritt hin gelegene Reaktionszone thermostatisiert werden. Alternativ kann die Gasphasenoxidation auch ohne Aufteilung in Temperaturzonen in beiden Reaktionszonen bei der gleichen Reaktionstemperatur durchgeführt werden.

Im allgemeinen wird die Umsetzung durch die Temperatureinstellung des Salzbades so gesteuert, daß in der ersten Zone der größte Teil des im Eduktgas enthaltenen aromatischen Kohlenwasserstoffs bei maximaler Ausbeute umgesetzt wird. Vorzugsweise wird der aromatische Kohlenwasserstoff in der ersten Reaktionszone nahezu vollständig bei maximaler Ausbeute umgesetzt.

Mit dem erfindungsgemäßen Verfahren werden, wie durch die nachfolgenden Beispiele belegt, die Nachteile der PSA-Verfahren und Katalysatoren des Standes der Technik auch bei hohen Beladungen des Eduktgasstroms mit o-Xylol und/oder Naphthalin von 80 g/Nm³ Gas und höher vermieden. Dies ist besonders überraschend, da die erfindungsgemäß anzuwendenden Katalysatoren relativ einfach zusammengesetzt sind und der Katalysator der ersten Reaktionszone, verglichen mit anderen Katalysatoren des Standes der Technik, einen sehr niedrigen Vanadiumoxidgehalt hat.

### Beispiele:

In allen Beispielen zur Katalysatorherstellung wurde die BET-Oberfläche nach der Methode von Brunauer et al, J. Am. Chem. Soc. 60, 309 (1938) bestimmt.

### Beispiel 1: Herstellung des Katalysators I

700 g Steatit-Ringe mit einem äußeren Durchmesser von 8 mm, einer Länge von 6 mm und einer Wandstärke von 1,5 mm wurden in einer Dragiertrommel auf 160°C erhitzt und mit einer Suspension aus 400 g Anatas (Analyse: Ti: 59,5 Gew.-%; S: 0,18 Gew.-%; P: 0,08 Gew.-%; Nb: 0,32 Gew.-%; K: 0,007 Gew.-%; Na: 0,04 Gew.-%; Fe: 0,002 Gew.-%; Zr: 0,003 Gew.-%; Pb: 0,003 Gew.-%; W: 0,02 Gew.-%) mit einer BET-Oberfläche von 21 m²/g, 30,7 g Vanadyloxalat, 2,60 g Cäsiumsulfat, 618 g Wasser und 128 g Formamid besprüht, bis das Gewicht der aufgetragenen Schicht 10,5 % des Gesamtgewichts des fertigen Katalysators entsprach. Die auf diese Weise aufgebrachte katalytisch aktive Masse, also die Katalysatorschale, enthielt 4 Gew.-% Vanadium (berechnet als V₂O₅), 0,4 Gew.-% Cäsium (berechnet als Cs) und 95,6 Gew.-% Titandioxid.

### Beispiel 2: Herstellung des Katalysators II

700 g Steatit-Ringe mit einem äußeren Durchmesser von 8 mm, einer Länge von 6 mm und einer Wandstärke von 1,5 mm wurden in einer Dragiertrommel auf 180°C erhitzt und mit einer Suspension aus 400 g Anatas (Analyse: Ti: 59,5 Gew.-%; S: 0,18 Gew.-%; P: 0,08 Gew.-%; Nb: 0,32 Gew.-%; K: 0,007 Gew.-%; Na: 0,04 Gew.-%; Fe: 0,002 Gew.-%; Zr: 0,003 Gew.-%; Pb: 0,003 Gew.-%; W: 0,02 Gew.-%) mit einer BET-Oberfläche von 21 m²/g, 57,6 g Vanadyloxalat, 14,4 g Antimontrioxid, 2,5 g Ammoniumhydrogenphosphat, 0,65 g Cäsiumsulfat, 618 g Wasser und 128 g Formamid besprüht, bis das Gewicht der aufgetragenen Schicht 10,5 % des Gesamtgewichts des fertigen Katalysators entsprach. Die auf diese Weise aufgebrachte katalytisch aktive Masse, also die Katalysatorschale, enthielt 0,15 Gew.-% Phosphor (berechnet als P), 7,5 Gew.-% Vanadium (berechnet als V₂O₅), 3,2 Gew.-% Antimon (berechnet als Sb₂O₃), 0,1 Gew.-% Cäsium (berechnet als Cs) und 88,75 Gew.-% Titandioxid.

### Beispiel 3: Herstellung des Katalysators III

700 g Steatit-Ringe mit einem äußeren Durchmesser von 8 mm, einer Länge von 6 mm und einer Wandstärke von 1,5 mm wurden in einer Dragiertrommel auf 160°C erhitzt und mit einer Suspension aus 400 g Anatas mit einer BET-Oberfläche von 21 m²/g, 30,7 g Vanadyloxalat, 3,33 g Ammoniumhydrogenphosphat, 2,60 g Cäsiumsulfat, 618 g Wasser und 128 g Formamid besprüht, bis das Gewicht der aufgetragenen Schicht 10,5 % des Gesamtgewichts des fertigen Katalysators entsprach. Die auf diese Weise aufgebrachte katalytisch aktive Masse, also die Katalysatorschale, enthielt 4,0 Gew.-% Vanadium (berechnet als V₂O₅), 0,2 Gew.-% Cäsium (berechnet als Cs), 0,2 Gew.-% Phosphor (berechnet als P) und 95,4 Gew.-% Titandioxid.

### Beispiel 4: Katalysatorsystem A

Es wurde in der-ersten Reaktionszone Katalysator I und in der zweiten Reaktionszone Katalysator II eingesetzt, deren katalytisch aktive Masse folgende Zusammensetzung in Gew.-%, bezogen auf das Gewicht der katalytisch aktiven Masse im fertigen Katalysator, hatte:

| | Katalysator I | Katalysator II |
|---|---|---|
| V₂O₅ | 4,0 | 7,5 |
| Sb₂O₃ | - | 3,2 |
| Cs | 0,4 | 0,1 |
| Phosphor (P) | - | 0,15 |
| Rest | Anatas | Anatas |

Ein Reaktionsrohr aus Stahl, von 3,5 m Länge und 25 mm Innendurchmesser, wurde im unteren Teil mit Katalysator II und im darüberliegenden Teil mit Katalysator I gefüllt. Die Füllhöhe des Katalysators II betrug 130 cm, die des Katalysators I 160 cm. Im oberen Teil war das Reaktionsrohr am Gaseingang in einer Länge von 50 cm frei von Katalysator. Das Reaktionsrohr wurde mit einem flüssigen Wärmeträgermedium (Salzbad) thermostatisiert, über das auch die Reaktionswärme abgeführt wurde.

Der Katalysatorträger bestand aus zylindrischen Ringen in den Abmessungen 6 mm (Höhe), 8 mm (Breite) und 5 mm (lichte Weite).

Das Reaktionsrohr wurde mit stündlich 4 m³ Luft von oben nach unten durchströmt, wobei die o-Xylol-Beladung 95 g/m³ Luft betrug. Die Reinheit des o-Xylols war 98,2 %ig. Bei einer Salzbadtemperatur von 350°C erreichte die hot spot-Temperatur im Katalysatorbett 430 bis 440°C. Der hot spot befand sich in ca. 40 bis 60 cm Tiefe der ersten Reaktionszone.

Das aus dem Reaktionsrohr austretende Reaktionsgas bestand in seinen organischen Bestandteilen zu 95,8 Gew.-% aus Phthalsäureanhydrid. Wesentliche Nebenprodukte waren Maleinsäureanhydrid (3,2 Gew.-%), Benzoesäure (0,51 Gew.-%), Phthalid (0,07 Gew.-%) und Citraconsäureanhydrid (0,34 Gew.-%).

Die gebildete PSA-Menge betrug 435 g/h, entsprechend einer PSA-Ausbeute von 83,5 mol-% bezogen auf 100 %iges o-Xylol. Das aus dem Reaktionsgas abgeschiedene Roh-PSA ließ sich in einer 2-stufigen Vakuumdestillation problemlos und ohne Vorbehandlung zu Rein-PSA der GC-Reinheit 99,9 % aufarbeiten. Die Schmelzfarbzahl betrug 5 bis 10 (APHA), die Hitzefarbzahl (90 Min. bei 250°C) 10 bis 20 (APHA).

Nach einjähriger Betriebszeit des Katalysators betrug die PSA-Ausbeute noch 82,7 mol-%.

Die für den Umweltschutz kritischen Stoffe o-Xylol bzw. Benzol waren im Reaktionsgas mit 31 mg/m³ bzw. 2,5 mg/m³ enthalten. Aufgrund ihrer geringen Konzentrationen brauchten diese Stoffe aus dem Abgas nicht entfernt bzw. abgereichert werden.

### Beispiel 5: Katalysatorsystem B

Es wurde in der ersten Reaktionszone Katalysator I und in der zweiten Reaktionszone Katalysator III eingesetzt, deren katalytisch aktive Masse folgende Zusammensetzung in Gew.-%, bezogen auf das Gewicht der katalytisch aktiven Masse im fertigen Katalysator, hatte:

| | Katalysator I | Katalysator III |
|---|---|---|
| V₂O₅ | 4,0 | 4,0 |
| Cs | 0,4 | 0,2 |
| Phosphor (P) | - | 0,2 |
| Rest | Anatas | Anatas |

Ein Reaktionsrohr aus Stahl, von 3,5 m Länge und 25 mm Innendurchmesser, wurde im unteren Teil mit Katalysator III und im darüberliegenden Teil mit Katalysator I gefüllt. Die Füllhöhe des Katalysators III betrug 130 cm, die des Katalysators I 170 cm.
Der obere Teil des Reaktionsrohres war am Gaseintritt in einer Länge von 40 cm leer.

Das Reaktionsrohr wurde mit einem flüssigen Wärmeträgermedium (Salzbad) thermostatisiert, über das auch die Reaktionswärme abgeführt wurde.

Der Katalysatorträger bestand aus zylindrischen Ringen in den Abmessungen 6,5 mm (Höhe), 7 mm (Breite) und 4 mm (lichte Weite).

Das Reaktionsrohr aus Beispiel 1 wurde mit stündlich 4 m³ Luft von oben nach unten durchströmt. Die o-Xylol-Beladung der Luft betrug dabei 85 g/m³. Die Reinheit des o-Xylols war 98,2 %. Bei einer Salzbadtemperatur von 355°C erreichte die hot spot-Temperatur 435 bis 445°C. Der hot spot befand sich in ca. 60 bis 70 cm Tiefe der ersten Reaktionszone.

Das aus dem Reaktionsrohr austretende Reaktionsgas bestand in seinen organischen Bestandteilen zu 95,9 Gew.-% aus Phthalsäureanhydrid. Wesentliche Nebenprodukte waren Maleinsäureanhydrid (2,87 Gew.-%), Benzoesäure (0,49 Gew.-%), Phthalid (0,18 Gew.%) und Citraconsäureanhydrid (0,35 Gew.-%).

Es wurden stündlich 388 g PSA gebildet, entsprechend einer PSA-Ausbeute von 83,2 mol-%, bezogen auf 100 %iges o-Xylol. Das aus dem Reaktionsgas gewonnene Roh-PSA ließ sich in einer 2-stufigen Vakuumdestillation problemlos und ohne Vorbehandlung zu Rein-PSA mit einer GC-Reinheit von 99,9 % aufarbeiten. Die Schmelzfarbzahl des Rein-Produktes betrug 5 bis 10 (APHA), die Hitzefarbzahl (90 Min. bei 250°C) 20 (APHA).

Nach 10-monatiger Betriebszeit des Katalysators betrug die PSA-Ausbeute noch 82,8 mol-%.

Die für den Umweltschutz problematischen Stoffe o-Xylol und Benzol lagen in vergleichbaren Konzentrationen wie in Beispiel 1 beschrieben vor. Eine Abreicherung aus dem Abgas war auch hier nicht erforderlich.

## Patentansprüche

1. Verfahren zur Herstellung von Phthalsäureanhydrid durch die katalytische Gasphasenoxidation von o-Xylol oder Naphthalin oder o-Xylol/Naphthalin-Gemischen mit einem molekularen Sauerstoff enthaltenden Gas an einem Schalenkatalysator aus einem inerten, nicht-porösen Trägermaterial, auf das eine Titandioxid und Vanadiumpentoxid enthaltende katalytisch aktive Masse schichtförmig aufgebracht ist, **dadurch gekennzeichnet, daß** man einen Katalysator dessen katalytisch aktive Masse zu 3 bis 6 Gew.-% aus Vanadiumpentoxid, berechnet als V₂O₅, zu 0,3 bis 0,5 Gew.-% aus einer Cäsiumverbindung, berechnet als Cs, und der Rest zu 100 Gew.-% aus Titandioxid in der Anatasmodifikation besteht, in An- oder Abwesenheit eines von diesem verschiedenen Schalenkatalysator zur katalytischen Gasphasenoxidation von o-Xylol oder Naphthalin oder o-Xylol/Naphthalin-Gemischen, verwendet und bei Anwesenheit eines solchen zweiten Katalysators, diesen in einer kombinierten Schüttung mit dem Katalysator obenstehender Zusammensetzung im Reaktor einsetzt.

2. Verfahren nach Anspruch 1, **dadurch gekennzeichnet, daß** man in einer ersten zum Gaseintritt in den Reaktor hin gelegenen Reaktionszone einen Schalenkatalysator einsetzt, dessen katalytisch aktive Masse zu 3 bis 6 Gew.-% aus Vanadiumpentoxid, berechnet als V₂O₅, zu 0,3 bis 0,5 Gew.-% aus einer Cäsiumverbindung, berechnet als Cs und der Rest zu 100 Gew.-% aus Titandioxid in der Anatasmodifikation besteht, und in einer zweiten zum Austritt der Reaktionsgase aus dem Reaktor hin gelegenen Reaktionszone einen Schalenkatalysator verwendet, dessen katalytisch aktive Masse zu 1 bis 10 Gew.-% aus Vanadiumpentoxid, berechnet als V₂O₅, zu 0 bis 10 Gew.-% aus Antimonoxid, berechnet als Sb₂O₃, zu 0,01 bis 0,3 Gew.-% aus einer Cäsium- und/oder Rubidiumverbindung, berechnet als Cs bzw. Rb, zu 0,01 bis 0,3 Gew.-% aus einer Phosphorverbindung, berechnet als P, und im Rest zu 100 Gew.-% aus Titandioxid in der Anatasmodifikation besteht.

3. Katalysator aus einer dünnen Schicht katalytisch aktiver Komponenten, die auf einem nicht-porösen Trägermaterial schalenförmig aufgebracht ist, dessen katalytisch aktive Masse zu 3 bis 6 Gew.-% aus Vanadiumpentoxid, berechnet als V₂O₅, zu 0,3 bis 0,5 Gew.-% aus einer Cäsiumverbindung, berechnet als Cs, und der Rest zu 100 Gew.-% aus Titandioxid in der Anatasmodifikation besteht.

## Claims

1. A process for preparing phthalic anhydride by catalytic gas-phase oxidation of o-xylene or naphthalene or o-xylene/naphthalene mixtures with a gas comprising molecular oxygen over a coated catalyst comprising an inert, nonporous support material on which a catalytically active composition comprising titanium dioxide and vanadium pentoxide is applied in layer form, wherein a catalyst whose catalytically active composition comprises from 3 to 6% by weight of vanadium pentoxide, calculated as V₂O₅, from 0.3 to 0.5% by weight of a cesium compound, calculated as Cs, and the remainder to 100% by weight of titanium dioxide in the anatase modification is used in the presence or absence of a coated catalyst, differing therefrom, for the catalytic gas-phase oxidation of o-xylene or naphthalene or o-xylene/naphthalene mixtures and, in the presence of such a second catalyst, the latter is used in a combined bed with the catalyst of the above composition in the reactor.

2. A process as claimed in claim 1, wherein the coated catalyst used in a first reaction zone nearest the gas inlet into the reactor is one whose catalytically active composition comprises from 3 to 6% by weight of vanadium pentoxide, calculated as V₂O₅, from 0.3 to 0.5% by weight of a cesium compound, calculated as Cs, and the remainder to 100% by weight of titanium dioxide in the anatase modification and the coated catalyst used in a second reaction zone nearest the outlet for the reaction gases from the reactor is one whose catalytically active composition comprises from 1 to 10% by weight of vanadium pentoxide, calculated as V₂O₅, from 0 to 10% by weight of antimony oxide, calculated as Sb₂O₃, from 0.01 to 0.3% by weight of a cesium and/or rubidium compound, calculated as Cs or Rb, from 0.01 to 0.3% by weight of a phosphorus compound, calculated as P, and the remainder to 100% by weight of titanium dioxide in the anatase modification.

3. A catalyst comprising a thin layer of catalytically active components applied in the form of a shell to a nonporous support material, wherein the catalytically active composition comprises from 3 to 6% by weight of vanadium pentoxide, calculated as V₂O₅, from 0.3 to 0.5% by weight of a cesium compound, calculated as Cs, and the remainder to 100% by weight of titanium dioxide in the anatase modification.

## Revendications

1. Procédé pour la préparation d'anhydride d'acide phtalique par oxydation catalytique en phase gazeuse de o-xylène ou de naphtaline ou de mélanges de o-xylène et de naphtaline par un gaz contenant de l'oxygène moléculaire, sur un catalyseur sous forme de coquille constitué d'un matériau de support inerte non poreux sur lequel une masse catalytiquement active contenant du dioxyde de titane et du pentoxyde de vanadium est appliquée en couches, **caractérisé en ce que** l'on utilise un catalyseur dont la masse catalytiquement active est constituée de 3 à 6 % en poids de pentoxyde de vanadium, pourcentage calculé en tant que V₂O₅, de 0,3 à 0,5 % en poids d'un composé de césium, pourcentage calculé en tant que Cs, le solde par rapport à 100 % étant constitué de dioxyde de titane dans sa variante anastase, en présence ou en l'absence d'un catalyseur en pellicule, différent de celui-ci, pour l'oxydation catalytique en phase gazeuse de o-xylène de naphtaline ou de mélanges de o-xylène et de naphtaline, et en cas de présence d'un tel deuxième catalyseur, ce dernier est utilisé dans le réacteur dans une combinaison en vrac avec le catalyseur présentant la composition ci-dessus.

2. Procédé selon la revendication 1, **caractérisé en ce que** dans une première zone de réaction située à l'entrée des gaz dans le réacteur, on utilise un catalyseur sous forme de coquille dont la masse catalytiquement active est constituée de 3 à 6 % en poids de pentoxyde de vanadium, pourcentage calculé en tant que V₂O₅, de 0,3 à 0,5 % en poids d'un composé de césium, pourcentage calculé en tant que Cs, le solde par rapport à 100 % en poids étant constitué de dioxyde de titane dans sa variante anastase, et dans une deuxième zone de réaction située à la sortie des gaz de réaction hors du réacteur, on utilise un catalyseur sous forme de coquille dont la masse catalytiquement active est constituée de 1 à 10 % en poids de pentoxyde de vanadium, pourcentage calculé en tant que V₂O₅, de 0 à 10 % en poids de monoxyde d'antimoine, pourcentage calculé en tant que Sb₂O₃, de 0,1 à 0,3 % en poids d'un composé de césium et/ou de rubidium, pourcentage calculé respectivement en tant que Cs ou Rb, de 0,01 à 0,3 % en poids d'un composé de phosphore, pourcentage calculé en tant que P, le solde par rapport à 100 % en poids étant constitué de dioxyde de titane dans sa variante anastase.

3. Catalyseur constitué d'un composant catalytiquement actif qui est appliquée en forme de coquille sur un matériau de support non poreux et dont la pâte catalytiquement active est constituée de 3 à 6 % en poids de pentoxyde de vanadium, pourcentage calculé en tant que V₂O₅, de 0,3 à 0,5 % en poids d'un composé de césium, pourcentage calculé en tant que Cs, le solde par rapport à 100 % en poids étant constitué de dioxyde de titane dans sa variante anastase.
